(19) Europäisches Patentamt
European Patent Office
Office européen des brevets

(11) Publication number: 0 255 937 A2

# (12) EUROPEAN PATENT APPLICATION

(21) Application number: 87111248.8

(51) Int. Cl.⁴: A61K 7/48

(22) Date of filing: 04.08.87

(30) Priority: 07.08.86 JP 186060/86

(43) Date of publication of application:
17.02.88 Bulletin 88/07

(84) Designated Contracting States:
DE FR GB

(71) Applicant: KYOWA HAKKO KOGYO CO., LTD.
6-1, Ohte-Machi Itchome
Chiyoda-ku Tokyo(JP)

Applicant: IWASE COSFA CO., LTD.
51, Dosho-machi 2-chome
Higashi-ku Osaka-shi Osaka-fu(JP)

(72) Inventor: Egi, Makoto
4845-4, Ami Ami-machi
Inashiki-gun Ibaraki-ken(JP)
Inventor: Shirahata, Kunikatsu
4-11-5, Iwadominami
Komae-shi Tokyo(JP)
Inventor: Sakai, Yasuo
7-15-12, Chuo Ami-machi
Inashiki-gun Ibaraki-ken(JP)
Inventor: Uji, Yoshitaka
14.3. Shimizu-cho
Neyagawa-shi Osaka-fu(JP)
Inventor: Shibayama, Hiroharu
8-14, Nakamichi 1-chome
Higashinari-ku Osaka-shi Osaka-Fu(JP)

(74) Representative: Casalonga, Axel et al
BUREAU D.A. CASALONGA - JOSSE
Morassistrasse 8
D-8000 Munich 5(DE)

## (54) Skin-treatment products.

(57) Disclosed are skin-treatment products which comprise an enzyme-treated phospholipid, an aqueous phase component and an oily phase component.

The skin-treatment products are safe, have high emulsification stability and excellent moisturizing effect and provide excellent feeling in use.

EP 0 255 937 A2

## Background of the Invention

The present invention relates to skin-treatment products which are widely utilized in various industrial fields such as cosmetics, drugs, over-the-counter drugs, toiletries, etc.

In recent years, many investigations have been made on emulsification and progress in novel emulsifiers and emulsifying technique has been remarkable. As a result, emulsions having very high stability have been widely utilized as skin-treatment products in various industrial fields. However, most of them are skin-treatment products prepared using nonionic, anionic, cationic or amphoteric surfactants as emulsifiers, and many consumers feel considerably uneasy about safety of these emulsifying agents. On the other hand, emulsions prepared using as an emulsifying agent higher fatty acid soap which is relatively safe and comfortable to use have also been used heretofore. However, such emulsions are naturally alkaline, and so they are irritative to skin and when they are applied to skin, undesirable whiteness does not readily disappear. In order to resolve these problems, use of phospholipids (lecithins) as emulsifiers has been noted (Japanese Published Unexamined Patent Application Nos. 47608/80, 70826/8l, 209207/82 and l404/84).

Skin-treatment products containing phospholipids have no problems in safety. However, because of the weak surface activity of phospholipids, special methods for emulsification should be used as in the references mentioned above or other surfactants must be used in combination with phospholipids.

Under the circumstances, it has been desired to develop skin-treatment products which are safe and have excellent moisturizing effect by improving the surface activity of conventional phospholipids.

## Summary of the Invention

The present invention provides skin-treatment products which comprise an enzyme-treated phospholipid, an aqueous phase component and an oily phase component.

The skin-treatment products of the present invention are safe, have high emulsification stability and excellent moisturizing effect and provide excellent feeling in use.

## Detailed Description of the Invention

The enzyme-treated phospholipids used in the present invention are obtained by treating phospholipids with hydrolase, for example, phospholipase A or phospholipase A and phospholipase D and contain lysophospholipids as effective components. As shown by the scheme below, lysophospholipids have a structure wherein either of the fatty acids bound to the hydroxy groups at the α-position and β-position of glycerol in phospholipids in an ester form is released by hydrolysis.

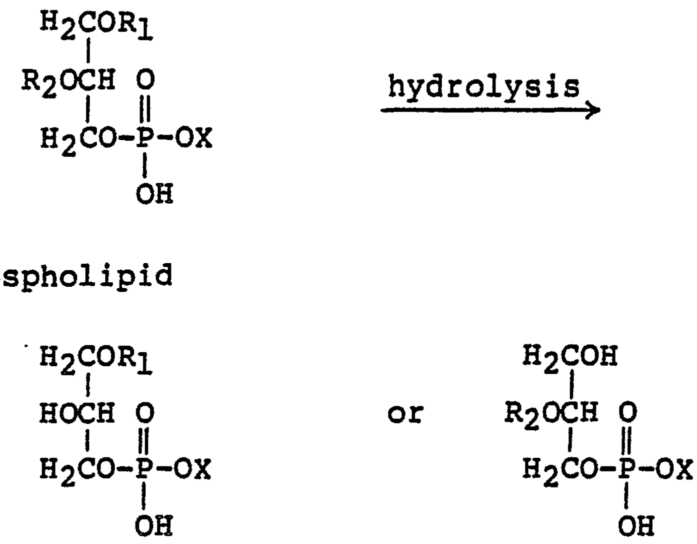


Phospholipid

or

Lysophospholipid

$R_1$, $R_2$: fatty acid acyl group

X: choline, ethanolamine, serine, inositol, etc.

As the method for preparing lysophospholipids, enzymatic methods which comprise treating phospholipids with phospholipase A are known [J. Am. Oil Chemists Soc., 53, 425-427 (1976), J. Sci. Food Agric., 32, 451-458 (1981), U.S.P. 4,478,866].

To prepare lysophospholipids according to the enzymatic method, soybean phospholipids or yolk phospholipids are usually used. Soybean and yolk phospholipids consist of phosphatidyl choline, phosphatidyl ethanolamine, phosphatidyl inositol, phosphatidyl serine, phosphatidic acid, etc. Ratios of the respective components vary depending upon the place of production, species, etc. of raw materials. As shown in Table 1, representative commercially available phospholipids (soybean lecithin) are greatly different from one another in composition. On the other hand, yolk phospholipids generally contain phosphatidyl choline as the principal component and phosphatidyl ethanolamine as the second component.

Table 1 Phospholipid composition of commercially available soybean lecithin (mol%)

| | PC | PE | PI | PS | PA |
|---|---|---|---|---|---|
| Product 1 | 41 | 34 | 19 | - | 6 |
| 2 | 33 | 29 | 24 | - | 14 |
| 3 | 45 | 19 | 11 | 25 | - |
| 4 | 11 | 19 | 41 | - | 29 |
| 5 | 33 | 32 | 21 | - | 14 |

PC : phosphatidyl choline
PE : phosphatidyl ethanolamine
PI : phosphatidyl inositol
PS : phosphatidyl serine
PA : phosphatidic acid

(Fette Seifen Anstrichmittel, vol. 81, No. 4, page 168, 1979)

Each phospholipid component is hydrolyzed by the treatment with phospholipase A. Therefore, the enzyme-treated phospholipids are mixtures of lysophospholipids unless they are purified. The conversion ratio of di-acyl phospholipids into lysophospholipids (hereinafter referred to as the conversion ratio) by enzymatic reaction varies depending upon the reaction conditions and reaction time. Even though residual di-acyl phospholipids are present in the enzyme-treated phospholipids, the products can be used in the present invention so long as lysophospholipids are contained at an effective concentration. In addition to the products obtained by treatment with phospholipase A alone, the lysophospholipids obtained by treating phospholipids with phospholipase A and phospholipase D simultaneously or sequentially and the enzyme-treated phospholipids containing suitable amounts of lysophospholipids obtained by controlling the reaction conditions and reaction time of phospholipase A and phospholipase D can be used in the present invention.

In general, commercially available soybean or yolk phospholipids contain impurities such as proteins, neutral lipids, inorganic substances, etc. In addition, as the result of the reaction with phospholipase A, free fatty acids are liberated from phospholipids. Further, liberation of the components such as choline, ethanolamine, inositol, serine, etc. is caused by the reaction with phospholipase D. Consequently, the enzyme-treated phospholipids contain these impurities.

These impurities can be removed by filtration; chromatography using cationic and anionic ion exchange resins; extraction with an organic solvent capable of dissolving lysophospholipids and phospholipids such as hexane, ether, petroleum ether, chloroform, kerosene, benzene, tetrahydrofuran, etc.; and extraction with an organic solvent like acetone suitable for the separation of free fatty acids and neutral lipids from phospholipids and lysophospholipids. Highly purified lysophospholipids or mixtures of lysophospholipids and phospholipids obtained by these purification methods singly or in combination can also be used in the present invention. These purified products have a light color and a less unpleasant smell so that they are more preferred for use as the ingredients of skin-treatment products.

An example of the purification method is given below. Phospholipids (soybean lecithin or yolk lecithin) are subjected to enzymatic reaction with phospholipase A or phospholipase A and phospholipase D, and the reaction solution is heated to stop the reaction. After the completion of reaction, insoluble matters in the reaction solution are filtered out and the filtrate is concentrated. Petroleum ether is added to the concentrate and the resulting solution is subjected to centrifugation to remove insoluble matters. Then, petroleum ether is removed by evaporation. The thus obtained phospholipids are washed with a sufficient amount of acetone to remove free fatty acids.

In case where high degree of purification is not required, extraction with petroleum ether can be omitted, and the thus obtained lysophospholipids or mixtures of lysophospholipids and phospholipids can also be used in the present invention.

In order to prevent oxidation of unsaturated fatty acid contained in phospholipids and lysophospholipids, the enzyme-treated phospholipids are hydrogenated after reduction.

The hydrogenation is carried out by ordinary catalytic hydrogenation which comprises adding palladium on carbon catalyst to the enzyme-treated phospholipids and stirring the mixture under a hydrogen atmosphere. It has become clear that the thus obtained enzyme-treated and hydrogenated phospholipids can be used in the present invention because they have a light color and a less unpleasant smell and their surface activity does not change by the hydrogenation to a large extent.

Previously hydrogenated phospholipids can also be used as raw materials for the enzyme reaction. Alternatively, the enzyme-treated phospholipids may also be purified to a high purity by the methods described above and then hydrogenated.

The enzyme-treated phospholipids contain l to l00 wt%, preferably 20 to l00 wt% of lysophospholipids. In case where lysophosphatidic acid is present in lysophospholipids contained in the enzyme-treated phospholipids, lysophosphatidic acid may be converted into a salt.

Examples of the salts include inorganic salts such as sodium salt, potassium salt, lithium salt, ammonium salt, calcium salt, magnesium salt, etc.; salts with basic amino acids such as lysine, arginine, histidine, ornithine, etc.; salts with basic oligopeptide containing these basic amino acids as constituent components; and salts with basic amines such as monoethanolamine, diethanolamine, triethanolamine, etc. The salts with these substances may be previously formed independently or may be formed during the preparation of the emulsion.

In the enzyme treatment of the present invention, the conversion ratio by phospholipase A is 20 to l00 mol%, preferably 50 to l00 mol%.

As the oily phase components used in the present invention, those conventionally used in the fields of cosmetics, over-the-counter drugs, pharmaceuticals, toiletries, etc. are appropriate. Examples of the oily phase components include liquid or semi-solid (greasy) hydrocarbons such as liquid paraffin, squalane, vaseline, etc.; ester oils such as octyldodecyl myristate, isopropyl palmitate, cetyl-2-ethyl hexanoate, glyceryl-tri-2-ethyl hexanoate, vitamin E acetate, vitamin A palmitate, vitamin C stearate, etc.; waxes such as solid paraffin, microcrystalline wax, ceresine wax, beeswax, spermaceti, etc.; vegetable fats and oils such as olive oil, soybean oil, safflower oil, rice bran oil, almond oil, camellia oil, jojoba oil, etc.; animal fats and oils such as tallow, lard, mink oil, turtle oil, etc.; higher alcohols such as cetanol, stearyl alcohol, cetostearyl alcohol, oleyl alcohol, octyldodecanol, behenyl alcohol, etc.; higher fatty acids such as lauric acid, myristic acid, palmitic acid, stearic acid, oleic acid, linoleic acid, linolenic acid, isostearic acid, etc.; and silicone oils such as dimethyl silicone, methylphenyl silicone, a cyclic silicone, etc. These substances can be used singly or as a mixture.

As the aqueous phase components used in the present invention, there are mentioned water; monovalent alcohols such as ethanol, propanol, isopropanol, butanol, etc.; polyvalent alcohols having two or more hydroxyl groups in the molecule thereof such as ethylene glycol, propylene glycol, l,3-butylene glycol, l,4-butylene glycol, hexylene glycol, glycerine, diglycerine, triglycerine, glucose, maltose, maltitol, sucrose, sorbitol, etc.; and other aqueous phase components which have been hitherto used in the production of cosmetics, over-the-counter drugs, pharmaceuticals, toiletries, etc. These substances can be used singly or as a mixture.

The emulsion of the present invention may also contain components other than these essential components; for example, various additives such as a pharmaceutical substance, a UV absorbant, a preservative and sterilizer, an antioxidant, a perfume, a coloring agent, a thickner, a stabilizer, etc. may also be formulated in the emulsion. In addition to nonionic, anionic, cationic and amphoteric surfactants currently used, synthetic or natural surfactants such as casein, sodium caseinate, saponin, etc. may also be used in combination, depending upon the purpose of use of the skin-treatment products.

The skin-treatment product of the present invention is an emulsion in which the aforesaid components are emulsified. It is desirable that the pH of the product is 3 to 8, because stability of the emulsion is poor when the pH is outside this range.

A preferred composition of the skin-treatment product comprises 0.05 to 30 wt%, preferably 0.l to l5 wt%, of the enzyme-treated phospholipid, l0 to 95 wt%, preferably l5 to 90 wt%, of the aqueous phase component, and 0.5 to 90 wt%, preferably l to 85 wt%, of the oily phase component.

Within the ranges described above, a good emulsion can be obtained. The skin-treatment products of the present invention can be obtained by emulsifying the components described above by conventional emulsifying means such as a homogenizer, etc.

Functions

The enzyme-treated phospholipids used in the present invention have a high surface activity and a good emulsifying action at low temperatures, as compared to commercially available phospholipids. The enzyme-treated phospholipids have an action of stabilizing the emulsion at high temperatures and an action of stabilizing the emulsion at low pH. Further, the skin-treatment products of the present invention are advantageous in that its stability is hardly affected by electrolytes such as sodium chloride, calcium chloride, etc.

Certain specific embodiments of the present invention are illustrated by the following representative examples and reference examples.

Example I and Reference Example I: pH stability of emulsion

Example I

To 400 g of crude hydrated lecithin (manufactured by True Lecithin Industry Co., Ltd.) having a water content of 55% was added 4 mℓ of 5N sodium hydroxide. The mixture was heated to 50°C and a suspension of I.2 g of pancreatin KM (manufactured by Mikuni Chemical Co., Ltd., I000 U/g) in 20 mℓ of water was added to the mixture with stirring. The reaction mixture was heated at 50°C overnight with stirring, concentrated under reduced pressure and dehydrated with a rotary evaporator. Diatomaceous earth was added to the system and the precipitates were removed by a pressure filtration device, whereby the enzyme-treated phospholipid having the conversion ratio of 80 mol% (lysophospholipid content: 36.4 wt%) was obtained.

I.0 g of the enzyme-treated phospholipid was mixed with I00 g each of citrate buffer solutions adjusted to pH 3.0, 5.0 and 7.0, respectively. While stirring with a homomixer (2,500 rpm), I00 g of squalane was added to the mixture and the mixture was emulsified for 30 seconds at 60°C to give emulsions. The pH stability (%) of each emulsion is shown in Table 2.

Evaluation of the pH stability of the emulsion:

After the emulsion was allowed to stand at 80°C for 4 hours, the volume of the whole solution phase (A) and the volume of the phase in an emulsion state (B) were measured and the emulsion rate (B/A × 100) was determined as an indication of an emulsifying force (stability of emulsion).

Reference Example I

Procedures similar to those in Example I were repeated except that lecithin (soybean phospholipid) manufactured by Ajinomoto Co., Inc. was used in place of the enzyme-treated phospholipid (conversion ratio: 80 mol%) of Example I. Evaluation of the stability of the emulsion was carried out in a similar manner as in Example I. The pH stability (%) of the emulsion is shown in Table 2.

Table 2. pH stability of emulsion

| | pH 7 | pH 5 | pH 3 |
|---|---|---|---|
| Example 1 | 87% | 85% | 80% |
| Reference Example 1 | 5% | 50% | 5% |

Example 2 and Reference Example 2:

Stability of emulsion at different temperatures

Example 2

While stirring I.0 g of the enzyme-treated phospholipid (conversion ratio: 80 mol%) used in Example I and I00 g of purified water with a homomixer (2,500 rpm), I00 g of squalane was added to the mixture, and the mixture was emulsified at 0°C for 30 seconds to give an emulsion.

Evaluation of the stability of the emulsion at different temperatures:

After the emulsion was allowed to stand at different temperatures (7, 37 and 80°C) for 4 hours, the stability was evaluated based on the calculation equation of the emulsion stability (%) in Example I. The results are shown in Table 3.

Reference Example 2

Procedures similar to those in Example 2 were repeated except that lecithin (soybean phospholipid) manufactured by Ajinomoto Co., Inc. was used in place of the enzyme-treated phospholipid (conversion ratio: 80 mol%) of Example I. Evaluation of the stability of the emulsion was carried out in a similar manner as in Example 2. The results are shown in Table 3.

Table 3. Stability of emulsion at different temperatures

| | Allowed to stand at 7°C | Allowed to stand at 37°C | Allowed to stand at 80°C |
|---|---|---|---|
| Example 2 | 87% | 75% | 70% |
| Reference Example 2 | 85% | 62% | 4% |

Example 3 and Reference Example 3:

Stability of emulsion to electrolytes

Example 3

Procedures similar to those in Example 2 were repeated except that 0.3% of calcium chloride was added as an electrolyte. Evaluation of the stability of the emulsion was carried out in a similar manner as in Example 2. The results are shown in Table 4.

Reference Example 3

Procedures similar to those in Reference Example 2 were repeated except that 0.3% of calcium chloride was added as an electrolyte. Evaluation of the stability of the emulsion was carried out in a similar manner as in Reference Example 2. The results are shown in Table 4.

Table 4. Stability of emulsion to electrolyte

| | Allowed to stand at 7°C | Allowed to stand at 37°C | Allowed to stand at 80°C |
|---|---|---|---|
| Example 3 | 82% | 75% | 60% |
| Reference Example 3 | 5% | 4% | 4% |

Example 4 Preparation of skin cream

The aqueous phase components and the oily phase components were added to monostearic acid polyoxyethylene sorbitan or to the enzyme-treated phospholipid having the conversion ratio of 70 mol% (lysophospholipid content: 31.9 wt%), which was prepared in a similar manner as in Example 1 except that the amount of the enzyme used was reduced by half, in formulation ratios shown in Table 5. The mixture was emulsified at 80°C for 5 minutes with a homomixer (4,000 rpm) and then cooled to 40°C to give skin cream. The formulation ratios are by wt% (the same shall apply hereinafter).

[Organoleptic test]

With respect to the skin cream obtained in Example 4 and control skin cream, organoleptic test was conducted by 20 adult female volunteers to examine moisturizing effect and feeling in use.

Designating "clearly effective", "somewhat effective" and "ineffective" as 2, 1 and 0, respectively, the total points of all the volunteers were determined and effectiveness (%) was calculated based on the following equation [effectiveness (%) = (total·points/40) × 100]. The obtained values are shown in Table 6 within parentheses together with the total points.

From the results shown in Table 6, it is evident that the skin cream prepared using the enzyme-treated phospholipid is much superior in both items for evaluation to the control skin cream prepared using monostearic acid polyoxyethylene sorbitan [20 E.O. (ethylene oxide: 20 moles)]. The control skin cream having a composition shown in Table 5 was prepared in a similar manner as in Example 4.

Table 5. Preparation of skin cream

| Components formulated | Example 4 | Control skin cream |
|---|---|---|
| | wt% | wt% |
| Monostearic acid polyoxyethylene sorbitan (20 E.O.) | - | 2.0 |
| Enzyme-treated phospholipid (conversion ratio: 70 mol%) | 2.0 | - |
| Aqueous phase: | | |
| Methyl p-oxybenzoate | 0.1 | 0.1 |
| ε-Aminocaproic acid | 0.1 | 0.1 |
| 1,3-Butylene glycol | 7.0 | 7.0 |
| Xanthane gum | 0.1 | 0.1 |
| Purified water | 25.7 | 25.7 |
| Oil phase: | | |
| Bleached white beeswax | 7.0 | 7.0 |
| Cetanol | 3.0 | 3.0 |
| Squalane | 35.0 | 35.0 |
| Octyldodecyl myristate | 20.0 | 20.0 |

Table 6. Evaluation by organoleptic test

| | Moisturizing effect | Feeling in use |
|---|---|---|
| Skin cream of Example 4 | 36 (90) | 38 (95) |
| Control skin cream | 12 (30) | 7 (18) |

Example 5 Hydrophilic ointment of the Japanese Pharmacopoeia

In accordance with the method for preparation of hydrophilic ointment of the llth Japanese Pharmacopoeia, components of Phase A shown in Table 7 were heated at 75°C to prepare a mixture. Phase B heated at 75°C was added to the mixture. While stirring, the mixture was cooled to give a hydrophilic ointment. The ointment was applied to volunteers with dry skin, and an excellent moisturizing effect was noted. Further, the ointment was compared with the ointment prepared according to the method of the Japanese

Pharmacopoeia, wherein sodium lauryl sulfate was used as an emulsifier. The ointment containing the enzyme-treated phospholipid was free from whiteness when applied to the skin and the feeling in use was also good. The enzyme-treated phospholipid was prepared in a similar manner as in Example I and provided for use.

Table 7. Hydrophilic ointment of the Japanese Pharmacopoeia

| | wt% |
|---|---|
| (Phase A) | |
| White vaseline | 25 |
| Stearyl alcohol | 22 |
| Propylene glycol | 12 |
| Enzyme-treated phospholipid (conversion ratio: 80 mol%, lysophospholipid content: 36.4 wt%) | 1.5 |
| Ethyl p-oxybenzoate | 0.025 |
| Propyl p-oxybenzoate | 0.015 |
| (Phase B) | |
| Purified water | 39.46 |

Example 6 Emolient cream

Phase B components in the formulation ratio shown in Table 8 were heated at 75°C and the resulting mixture was added to Phase A heated at the same temperature with stirring. After the mixture was treated with a homomixer (4,000 rpm) for 5 minutes, the system was cooled to 40°C and then Phase C was added thereto. The resulting mixture was cooled to room temperature to give emolient cream. This emolient cream had excellent moisturizing effect and provided excellent feeling in use.

Table 8. Emolient cream

| | wt% |
|---|---|
| (Phase A) | |
| ε-Aminocaproic acid | 0.1 |
| Purified water | 56.05 |
| Purified enzyme-treated phospholipid* (conversion ratio: 80 mol%, lysophospholipid content: 73.7 wt%) | 2.5 |
| Glycerine | 8.0 |
| (Phase B) | |
| Squalane | 13.0 |
| Jojoba oil | 2.0 |
| Octyldodecyl myristate | 5.0 |
| Bleached white beeswax | 3.0 |
| Behenyl alcohol | 3.0 |
| Stearic acid | 6.0 |
| Methyl p-oxybenzoate | 0.3 |
| (Phase C) | |
| Coix extract | 0.5 |
| Chamomile extract | 0.5 |
| Perfume | 0.05 |

* The phospholipid was obtained by washing the enzyme-treated phospholipid (conversion ratio: 80 mol%) prepared as in Example 1 with a 10-fold amount of acetone, drying the washed phospholipid under reduced pressure and then grinding the dried product into a powder.

Example 7 Emolient cream

Emolient cream was prepared in a similar manner as in Example 6 except that hydrogenated enzyme-treated phospholipid was used in the same amount in place of the purified enzyme-treated phospholipid used in Phase A in Example 6. This emolient cream had excellent moisturizing effect and provided excellent feeling in use.

The hydrogenated enzyme-treated phospholipid was prepared by dissolving the purified enzyme-treated phospholipid powder (conversion ratio: 80 mol%, lysophospholipid content: 73.7 wt%) used in Example 6 in tetrahydrofuran at a concentration of 20% (W/V), adding 5% palladium on carbon catalyst to the solution in an amount of 5% (W/W) based on the powder, and subjecting the solution to hydrogenation at 50°C under 10 $kg/cm^2$ for 12 hours in a hydrogen atmosphere. After the catalyst was separated from the hydrogenated product, the solvent was removed by evaporation and the resulting powder was used as the hydrogenated enzyme-treated phospholipid. The iodine value of the enzyme-treated phospholipid was lowered from 60 to 30 by the hydrogenation.

Example 8 Milky lotion

Phase B components in the formulation ratio shown in Table 9 were heated at 75°C. To the resulting mixture were added Phase A and Phase C heated at the same temperature with stirring in this order. The mixture was treated for 5 minutes with a homomixer (4,000 rpm) and then cooled to 40°C. Phase D was added and the mixture was cooled to room temperature to give a milky lotion. This milky lotion exhibited excellent moisturizing effect and provided excellent feeling in use.

Table 9. Milky lotion

| | wt% |
|---|---|
| (Phase A) | |
| Purified enzyme-treated phospholipid* (conversion ratio: 50 mol%, lysophospholipid content: 41.2 wt%) | 4.0 |
| 1,3-Butylene glycol | 3.0 |
| L-Arginine | 0.1 |
| Purified water | 74.15 |
| (Phase B) | |
| Squalane | 5.0 |
| Jojoba oil | 1.0 |
| Natural vitamine E | 0.5 |
| Methyl p-oxybenzoate | 0.2 |
| (Phase C) | |
| Carboxy vinyl polymer | 10.0 |
| (Phase D) | |
| Perfume | 0.05 |
| Plant extract | 2.0 |

* The phospholipid was prepared in a similar manner as in Example 1 except that the amount of the enzyme used was reduced by half and the reaction time was 10 hours.

Example 9 Milky lotion

Milky lotion having the same composition as in Example 8 was prepared in a similar manner as in Example 8 except that the enzyme-treated phospholipid (conversion ratio: 75 mol%, lysophospholipid content: 52.5 wt%) prepared using yolk lecithin (manufactured by Q.P. Corporation) in a similar manner as in Example I was used as the enzyme-treated phospholipid.

This milky lotion was superior to control milky lotion in moisturizing effect and feeling in use. The control milky lotion was prepared in a similar manner as in Example 8 except that yolk lecithin (manufactured by Q.P. Corporation) was used in place of the enzyme-treated phospholipid.

**Claims**

I. A skin-treatment product which comprises an enzyme-treated phospholipid, an aqueous phase component and an oily phase component.

2. A skin-treatment product according to claim I, wherein the phospholipid is soybean lecithin or yolk lecithin.

3. A skin-treatment product according to claim I, wherein the contents of the enzyme-treated phospholipid, the aqueous phase component and the oily phase component are 0.05 to 30 wt%, I0 to 95 wt% and 0.5 to 90 wt%, respectively.

4. A skin-treatment product according to claim I, wherein the enzyme-treated phospholipid contains I to I00 wt% lysophospholipid.

5. A skin-treatment product according to claim I, wherein the enzyme-treated phospholipid is obtained by treating phospholipid with phospholipase A singly or phospholipase A and phospholipase D.

6. A skin-treatment product according to claim 5, wherein a conversion ratio by phospholipase A is 20 to I00 mol%.